# EUROPEAN PATENT APPLICATION

(11) **EP 4 279 585 A1**
(43) Date of publication of application: **22.11.2023**
(21) Application number: 21919042.8
(22) Date of filing: 29.11.2021
(51) Int. Cl.: C12N 5/10, C12N 15/62, C12N 15/13, C12N 15/867, C07K 16/28, A61K 39/395, A61P 35/02

(54) **CD7-TARGETED ENGINEERED IMMUNE CELL, CHIMERIC ANTIGEN RECEPTOR, CD7 BLOCKING MOLECULE AND USE THEREOF**

(30) Priority: 12.01.2021 CN 202110036169
(71) Applicant: Shanghai Yake Biotechnology Ltd., Shanghai 200438 (CN)
(72) Inventor: CHANG, Alex Hongsheng, Shanghai 200438 (CN)
(74) Representative: TBK
(86) International application number: PCT/CN2021/133817
(87) International publication number: WO 2022/151851

(57) **Abstract**

A CD7-targeted engineered immune cell, a chimeric antigen receptor, a CD7 blocking molecule and the use thereof. A natural ligand of human CD7 is used for substituting for an antibody sequence to serve as an antigen recognition domain of a CD7-specific CAR-T or CAR-NK cell. The advantage of using human CD7 as the antigen recognition domain in the CD7-specific CAR is that cellular and humoral reactions produced by a host can be prevented, thereby achieving long-term durability and better efficacy of the CAR-T cell.

## Description

This application claims the benefit of CN Application No. 202110036169.2, filed January 12, 2021, the full disclosure of which is incorporated herein by reference in its entirety for all purposes.

### Technical Field

The present invention relates to the field of cell immunotherapy technology, specifically to a CD7-targeted immunotherapy method, especially to a CD7-targeted engineered immune cell, a chimeric antigen receptor, a CD7 blocking molecule and the use thereof.

### Background Art

T-cell malignancies are a group of diseases with highly heterogeneous clonal growth and T-cell dysfunction, mainly divided into T-cell lymphomas (TCLs) and T-cell leukemias, with mature and precursor subtypes. Such diseases represent a class of extremely malignant hematological cancers with high recurrence and mortality rates in both children and adults, and there is currently no effective or targeted treatment. Although there are multiple chemotherapy regimens available in the prior art, less than 50% of adults and 75% of children with T-cell acute lymphoblastic leukemia (T-ALL) can survive for more than 5 years. For patients who relapse after initial treatment, salvage chemotherapy regimens may induce remission in only 20%-40% of cases. In patients with relapsed or chemotherapy-refractory T-cell malignancies, the treatment prognosis is poor, and effective and tolerable treatments are limited. For 10%-50% of patients who achieve a complete remission (CR) after salvage chemotherapy, the only treatment option remains allogeneic stem cell transplantation (ASCT). However, ASCT has a cure rate that remains at 30% or lower, and not all CR patients are eligible for transplantation. Other T-cell malignancies, comprising cutaneous and peripheral T-cell lymphomas (CTCL and PTCL, respectively), have a lower initial response rate to chemotherapy, and even in responsive patients, the progression-free survival rate remains at 40%-50%. Therefore, despite progress in the treatment of T-cell malignancies, new targeted treatment strategies are still needed to improve prognosis, especially for relapsed and refractory patients. In addition to T-cell malignancies such as T-ALL and T-NHL, it must be recognized that effective treatment and targeted treatment strategies are still lacking for other CD7+ hematological malignancies, such as 20%-30% of acute myeloid leukemia (AML) and the vast majority of NK and NKT lymphomas.

Chimeric antigen receptor T (CAR-T) cell is one of the most promising tumor immunotherapy methods, which can result in a significant response rate in patients with B lymphocyte malignancies. Therefore, CAR-T cells targeting CD19 (an antigen widely expressed in B-cell leukemia and lymphoma) have become the first approved T-cell therapy for cancer. The success of CD 19 CAR-T cells in treating relapsed and refractory B-cell malignancies has contributed to more widespread use thereof in other tumors. Given the similarity between B lymphocyte and T lymphocyte malignancies, it seems simple to extend CAR-T cell therapy to these diseases. However, CAR-T cell therapy for T cell malignancies has been demonstrated difficult to develop and implement. This is mainly due to the fact that simultaneous expression of target antigens on engineered T cells can lead to CAR-T cells fratricide during preparation; in addition, the clearance of normal peripheral blood T cells after CAR-T cells being infused can lead to severe immune deficiency.

CD7 is a transmembrane glycoprotein commonly expressed on most peripheral blood T cells and NK cells (NK cells are natural killer cells, derived from bone marrow lymphoid stem cells, and their differentiation and development depend on the bone marrow and thymus microenvironment, which are mainly distributed in bone marrow, peripheral blood, liver, spleen, lung and lymph nodes; unlike T and B cells, NK cells are a class of lymphocytes that can non-specifically kill tumor cells and virus-infected cells without prior sensitization) and the precursor cells thereof, which acts as a co-stimulatory protein that assists T cell activation and interacts with other immune subsets. More than 95% of lymphoblastic leukemias and lymphomas, as well as some of the peripheral T-cell lymphomas, express CD7. In mouse animal models, CD7-deficient T cells exhibit undisturbed development, homeostasis, and protective functions to a large extent. Since CD7 has no significant impact on the function of peripheral blood T cells, it is a promising target for CAR-T cell therapy. CD7 has been evaluated as a target for monoclonal antibody (mAb) in patients with T cell malignancies treated with immunotoxins. The conjugates of this monoclonal antibody did not produce severe CD7-related toxic and side effects, but the anti-tumor response was not significant, possibly due to the limited activity of murine-derived antibodies for the treatment in patients.

In the prior art, the progress on the study of targeted CD7 therapy is as follows:
i) CD7-CAR-T cell therapy; T cells expressing chimeric antigen receptor (CAR) represent a promising tumor immunotherapy method. This targeted therapy has shown great potential in achieving remission and even long-term survival without relapse in patients with B-cell leukemia and lymphoma. Several study groups have recently reported progress on the study of CD7-specific CAR-T cell therapy in preclinical models of T-cell malignancies. In all of these studies, the expression of CD7-CAR on T cells led to severe fratricide, preventing CAR-T cells from expanding in vitro.

The expression of target-specific CAR on transduced T cells can result in the activation of CAR receptors due to sustained ligand binding, which can lead to the behavior of fratricide among transduced T cells and accelerate terminal differentiation of cells so that they cannot survive in vivo for a long time. Since CD7 is a pan-T cell antigen and expressed on most T cells, severe fratricide may occur during preparation of CD7 antigen-specific CAR-T cells, resulting in CAR-T cells being unable to effectively expand. At present, there are two strategies to reduce this behavior of fratricide: 1) using genome editing tools to knock out the gene of CD7 target antigen; 2) preventing the CD7 protein from being transported to the cell surface during intracellular expression by means of anchoring the CD7 binding domain at the endoplasmic reticulum. Both methods can effectively reduce the expression of CD7 on the cell surface and minimize the behavior of fratricide among CD7-targeted CAR-T cells. Importantly, the absence of CD7 does not affect the proliferation and short-term effector function of CAR-T cells, allowing functional CAR-T cells with high anti-tumor activity to expand.

After removing CD7 from the cell surface, CD7-CAR-T cells have strong anti-tumor activity against primary CD7-positive T-ALL and lymphoma in vitro and in vivo. CD7-CAR-T cells can also kill peripheral blood CD7-positive T cells and NK cells, indicating that these cell subsets will also be targets for CD7-CAR-T cells.

One risk of using autologous cells for adoptive cell therapy is that malignant cells from peripheral blood are also inadvertently genetically modified. The use of CAR gene-modified malignant T cells, allowing them to undergo gene editing to reduce the expression of target molecules in certain cases, may pose a real risk, and therefore this needs to be explained in the informed consent form when using these therapies. However, considering the poor survival and amplification of malignant tumor cells during manufacturing, these unnecessary gene modifications on malignant cells should occur at a lower frequency. In addition, in the absence of gene editing to reduce the expression of target antigen, malignant tumor cells expressing target antigens may be cleared by CAR-T cells through the behavior of fratricide after being transduced and before being infused into the patient's body. Meanwhile, allogeneic CD7-CAR-T cells produced from healthy donors can be used to treat relapsed and refractory T-cell malignancies so as to achieve the goal of bridging to hematopoietic stem cell transplantation, or to treat T-cell malignancies that relapse after hematopoietic stem cell transplantation in patients. If the side effects of the graft-versus-host can be properly controlled, allogeneic CD7-CAR-T cell therapy has the advantage of producing graft-versus-leukemia effects in addition to being supplied with T cells by a healthy donor. Using off-the-shelf allogeneic T cells or NK cells as a vehicle can completely avoid the risk of genetically modifying malignant cells. Since these cell products will be produced by a healthy donor, the use of patient-derived T cells, which are often functionally impaired due to long-term exposure to suppressive tumor microenvironments or as a result of prior intensive therapy, can be avoided.
ii) CD7-targeted immunotoxins; CD7 is expressed at high density (about 60,000 mol/cell) on T cells, and CD7 is rapidly internalized even when bound by a monovalent antibody fragment. Therefore, CD7 is an ideal target antigen for immunotoxin-mediated therapy against T cell tumors. Currently, anti-CD7 immunotoxins are mainly formed by conjugating an anti-CD7 monoclonal antibody with toxins. An anti-CD7 immunotoxin formed by conjugating a mouse anti-human CD7 monoclonal antibody (WTI) with ricin A has been used to clear tumor cells in vitro for autologous bone marrow transplantation in patients with T cell malignancies. The immunotoxin DA7 was constructed by chemically linking mouse IgG2b anti-CD7 (3AlE) monoclonal antibody with deglycosylated ricin A chain, and has been used to treat human T-ALL in SCID animal models, suggesting its potential therapeutic effect on T-cell leukemia with poor prognosis. In Phase I clinical trials of DA7, objective clinical responses were achieved at its maximum tolerated dose despite limitations of instability and vascular toxicity. The anti-human CD7 monoclonal antibody TH69 has also been used to construct recombinant immunotoxins, wherein the antibody is formed by linking single-chain antibody fragments (scFv) with truncated Pseudomonas exotoxin A fragments through genetic recombination.
iii) Gene knockout; Targeted gene editing of T cells has been demonstrated to be clinically feasible for adoptive immunotherapy. Gene knockout of the HIV co-receptor CCR5 in CD4-positive T cells is carried out by using zinc finger nucleases, so that these cells become resistant to HIV infection, and CCR5-negative T cells can be implanted and persist in HIV-infected patients. The T cell receptor (TCR) genes in CD19 CAR-T cells are knocked out by using TALENs, so that third-party T cells can successfully treat patients with B-cell leukemia, which is an encouraging milestone for "universal" off-the-shelf T cell products and greatly reduces the risk of graft-versus-host disease. In this study, the CD52 gene was also knocked out in the CAR-T cells that were infused, making these cells resistant to alemtuzumab. Recently, the CRISPR/Cas9 system has been used to knock out CD7 or knock out both CD7 and T cell receptor α chain, and this new gene editing system can quickly and effectively knock out target genes in T cells.
iv) Technology for blocking intracellular protein expression; In many biochemical and immune systems, it is often difficult to determine the function of certain specific molecules, but there is a method that can inhibit gene expression thereof and look for its functional effects. Gene knockout can be achieved through homologous recombination or gene editing technologies such as Zinc Finger Nucleases, TALENs, and CRISPR/Cas9 developed in recent years. Although these technologies are very powerful, there are many shortcomings or technical difficulties in practice. Therefore, there is a great need to develop a simple and effective technology to remove molecules in a controllable way.

A large amount of studies has shown that using intracellular antibodies can target and inactivate important molecules in cells. By constructing heavy and light chains of antibodies to transfect mammalian cells with simple modifications on their lead sequences, the antibody sequences can be anchored at three main intracellular regions: endoplasmic reticulum (ER)/Golgi complex, cytoplasm, and nucleus. A full IgG was used in the initial study on intracellular antibodies, but recent studies have focused on Fab' and single-chain antibodies (scFv). Single-chain antibody molecules are very small (about 30 kDa) and are constructed by connecting the variable regions of heavy and light chains via short peptide sequences. These constructs have many advantages as intracellular antibodies, mainly because it is not necessary to combine two independent antibody chains to form an antigen-binding site.

So far, studies have shown that intracellular antibodies can be used to prevent the processing of HIV gp160 to gp130 in the endoplasmic reticulum of cells, block the expression of IL-2 receptor α chain, inhibit cytoplasmic enzymes in yeast, oocytes from Xenopus laevis, and human T-cell lines, and inhibit virus activity in transgenic plants. A study by Marasco and his colleagues shows that single-chain antibodies can work in ER. A study by Greenman et al. shows that intracellular single-chain antibodies can be used to prevent the expression of cell membrane proteins, wherein a high-affinity monoclonal antibody that can bind to the T lymphocyte surface antigen CD2 and an ER retention signal (KDEL) are used.

The antigen recognition domain is an important component of the CAR structure, responsible for binding to tumor cell surface antigens. Currently, the antigen recognition domain for CD7-specific CAR-T cell therapy is mostly composed of variable regions of mouse or alpaca-derived antibodies. Once infused into the patient's body, CAR-T cells carrying alloantigen recognition domains can induce strong host cell and humoral responses. The infused CAR-T cells are rapidly cleared by the host immune system, which seriously affects the persistence of CAR-T cells in the body, and ultimately leads to refractory and relapsed diseases. In the present invention, natural human CD7 ligands are used as the antigen recognition domain of CAR, which has the advantage that the CD7-CAR-T cells constructed thereby do not induce host immune responses and can survive in the body for a long time, thereby achieving better therapeutic effects.

### Summary of the Invention

In view of the defects in the prior art., the objective of the present invention is to provide a CD7-targeted engineered immune cell, a chimeric antigen receptor, a CD7 blocking molecule and the use thereof.

The objective of the present invention is realized by the following solutions.

The first aspect of the present invention provides an engineered immune cell comprising a polynucleotide sequence encoding a chimeric antigen, wherein the chimeric antigen receptor comprises a CD7-targeted antigen recognition domain.

In another preferred example, the chimeric antigen receptor further comprises a hinge and transmembrane domain, an intracellular co-stimulatory domain, and an intracellular primary stimulatory domain.

In another preferred example, the hinge and transmembrane domain in the chimeric antigen receptor is selected from at least one group of the following amino acid sequences: amino acid sequences of α and β chains from T cell receptor, CD3δ, CD3ε, CD3γ, CD3ζ chain, CD4, CD5, CD8α, CD8β, CD9, CD16, CD22, CD28, CD32, CD33, CD34, CD35, CD37, CD45, CD64, CD80, CD86, CD137, ICOS, CD154, FAS, FGFR2B, OX40, or VEGFR2.

In another preferred example, the hinge and transmembrane domain in the chimeric antigen receptor is derived from CD8α.

In another preferred example, the amino acid sequence of the hinge and transmembrane domain CD8α in the chimeric antigen receptor is as shown in SEQ ID NO. 1.

In another preferred example, the intracellular co-stimulatory domain in the chimeric antigen receptor is selected from at least one of the following: CD2, CD4, CD5, CD8α, CD8β, CD27, CD28, CD30, CD40, 4-1BB (CD137), ICOS, OX40, LIGHT (CD258) or NKG2C.

In another preferred example, the intracellular co-stimulatory domain in the chimeric antigen receptor is derived from 4-1BB.

In another preferred example, the amino acid sequence of the intracellular co-stimulatory domain 4-1BB in the chimeric antigen receptor is as shown in SEQ ID NO. 2.

In another preferred example, the hinge and transmembrane domain and intracellular co-stimulatory domain in the chimeric antigen receptor are derived from CD28.

In another preferred example, the amino acid sequence of the hinge and transmembrane domain and the intracellular co-stimulatory domain CD28 in the chimeric antigen receptor is as shown in SEQ ID NO. 3.

In another preferred example, the intracellular primary stimulatory domain in the chimeric antigen receptor is selected from at least one of the following: CD3δ, CD3ε, CD3γ, CD3ζ, FcRβ, FcRy, CD5, CD66, CD22, CD79a or CD79b.

In another preferred example, the intracellular primary stimulatory domain in the chimeric antigen receptor is derived from CD3ζ.

In another preferred example, the amino acid sequence of the intracellular primary stimulatory domain CD3ζ in the chimeric antigen receptor is as shown in SEQ ID NO. 4.

In another preferred example, the CD7-targeted antigen recognition domain in the chimeric antigen receptor is a part or all of the sequences of a human CD7-L extracellular domain, or has at least 90% sequence identity to the human CD7-L extracellular domain, and the amino acid sequence of the human CD7-L extracellular domain is as shown in SEQ ID NO. 5.

In another preferred example, the engineered immune cell further comprises a CD7 blocking molecule, which comprises a CD7 binding domain and an intracellular anchoring domain, and can prevent the transport and expression of the CD7 protein on the cell surface.

In another preferred example, the CD7 blocking molecule prevents the CD7 protein from being transported to the cell surface specifically by connecting a CD7 binding domain to an intracellular anchoring domain.

In another preferred example, the intracellular anchoring domain is an amino acid sequence of an endoplasmic reticulum retention domain, a Golgi complex retention domain, or a proteasome localization domain.

In another preferred example, the intracellular anchoring domain is an endoplasmic reticulum retention domain.

In another preferred example, the amino acid sequence of the intracellular anchoring domain - endoplasmic reticulum retention domain, is as shown in SEQ ID NO. 6 or SEQ ID NO. 7.

In another preferred example, the CD7 binding domain is a part or all of the sequences of a human CD7-L extracellular domain, or has at least 90% sequence identity to the human CD7-L extracellular domain, and the amino acid sequence of the human CD7-L extracellular domain is as shown in SEQ ID NO. 5.

In another preferred example, the CD7 binding domain is a scFv of an anti-CD7 monoclonal antibody TH69.

In another preferred example, the CD7-targeted antigen recognition domain in the chimeric antigen receptor is a scFv of an anti-CD7 monoclonal antibody TH69, or has at least 90% sequence identity to the scFv of the anti-CD7 monoclonal antibody TH69, and the amino acid sequence of the scFv of the anti-CD7 monoclonal antibody TH69 is as shown in SEQ ID NO. 8.

In another preferred example, the engineered immune cells further comprise a CD7 blocking molecule, which can prevent the transport and expression of the CD7 protein on the cell surface.

In another preferred example, the CD7 blocking molecule prevents the CD7 protein from being transported to the cell surface by connecting a CD7 binding domain to an intracellular anchoring domain.

In another preferred example, the intracellular anchoring domain is an amino acid sequence of an endoplasmic reticulum retention domain, a Golgi complex retention domain, or a proteasome localization domain.

In another preferred example, the CD7 binding domain is a part or all of the sequences of a human CD7-L extracellular domain, or has at least 90% sequence identity to the human CD7-L extracellular domain, and the amino acid sequence of the human CD7-L extracellular domain is as shown in SEQ ID NO. 5.

In another preferred embodiment, the engineered immune cells is deprived of the gene expression of CD7 through gene knockout technology.

In another preferred example, the gene editing tool used for the knockout technology is TALENs or CRISPR/cas9.

In another preferred example, the cell is a T cells, a γδT cell, a NK or NKT cell, and a T cell, a γδT cell, a NK or NKT cell that induces differentiation of a pluripotent stem cell (iPSC).

The second aspect of the present invention provides a CD7-targeted antigen recognition domain in the chimeric antigen receptor, wherein the sequence of the antigen recognition domain comprises a part or all of the sequences of a human CD7-L extracellular domain, or has at least 90% sequence identity to the human CD7-L extracellular domain, and the amino acid sequence of the human CD7-L extracellular domain is as shown in SEQ ID NO. 5.

The third aspect of the present invention provides the use of the CD7-targeted antigen recognition domain as described above in the preparation of an immunotoxin for CD7-positive hematological malignancies.

The fourth aspect of the present invention provides a nucleic acid molecule encoding the CD7-targeted antigen recognition domain in the chimeric antigen receptor as described above.

In another preferred example, the nucleic acid molecule encoding the CD7-targeted antigen recognition domain in the chimeric antigen receptor is CD7BB-002, and the nucleotide sequence of CD7BB-002 is as shown in SEQ ID NO. 9.

The fifth aspect of the present invention provides a recombinant vector comprising a nucleic acid molecule encoding the CD7-targeted antigen recognition domain in the chimeric antigen receptor as described above.

In another preferred example, the vector is selected from a retrovirus, a lentivirus or a transposon.

The sixth aspect of the present invention provides the use of the recombinant vector as described above in the preparation of an engineered immune cell.

The seventh aspect of the present invention provides a CD7 blocking molecule for preparing the engineered immune cells as described above, wherein the CD7 binding domain of the CD7 blocking molecule comprises a part or all of the sequences of the human CD7-L extracellular domain, or is a protein having at least 90% sequence identity to the human CD7-L extracellular domain.

The eighth aspect of the present invention provides a nucleic acid sequence encoding the CD7 blocking molecule as described above.

In another preferred example, the nucleic acid sequence of the CD7 blocking molecule is CD7-L-ER2.1, and the nucleotide sequence of CD7-L-ER2.1 is as shown in SEQ ID NO. 10.

The ninth aspect of the present invention further provides a CD7 blocking molecule for preparing the engineered immune cells as described in the first aspect, wherein the CD7 binding domain of the CD7 blocking molecule is a scFv of an anti-CD7 monoclonal antibody TH69, or a protein having at least 90% sequence identity to the scFv of the anti-CD7 monoclonal antibody TH69.

The tenth aspect of the present invention provides a nucleic acid sequence encoding the CD7 blocking molecule as described in the ninth aspect. The coding sequence of the nucleic acid molecule of the CD7 blocking molecule is TH69-ER2.1, and the nucleotide sequence of TH69-ER2.1 is as shown in SEQ ID NO. 11.

The eleventh aspect of the present invention provides a recombinant vector, comprising the nucleic acid sequence encoding the CD7 blocking molecule provided in the eighth or tenth aspect.

In another preferred example, the vector is selected from a retrovirus, a lentivirus or a transposon.

The twelfth aspect of the present invention provides the use of the recombinant vector provided in the eleventh aspect in the preparation of an engineered immune cell.

The thirteenth aspect of the present invention provides a nucleic acid molecule encoding the CD7-targeted antigen recognition domain in the chimeric antigen receptor as described in the second aspect and the CD7 blocking molecule (the CD7 blocking molecule provided in the seventh or ninth aspect).

The fourteenth aspect of the present invention provides a recombinant vector, comprising the nucleic acid molecule as described in the thirteenth aspect.

In another preferred example, the vector is selected from a retrovirus, a lentivirus, or a transposon.

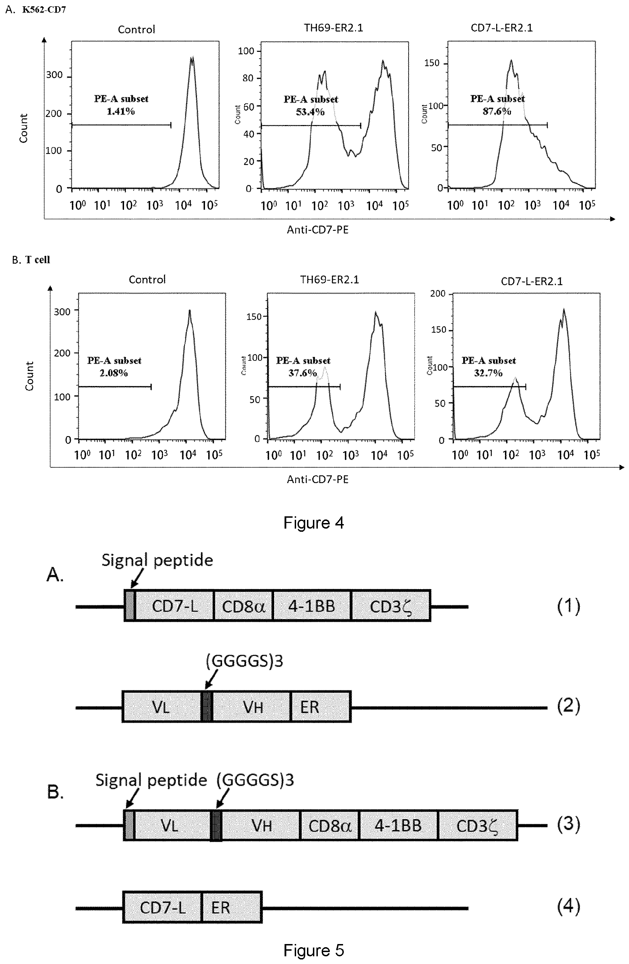

In another preferred example, the recombinant vector comprises the following sequence: CD7BB-BL4-002 or CD7BB-BL6-002, and the schematic diagram of the vector sequence of the recombinant vector CD7BB-BL4-002 or CD7BB-BL6-002 is as shown in A and B of Figure 5, respectively.

In another preferred example, the recombinant vector CD7BB-BL4-002 is formed by connecting CD7BB-002 to TH69-ER2.1 via T2A. The structural schematic diagram of the recombinant vector is as shown in (1) and (2) in A of Figure 5, and the amino acid sequence of T2A is as shown in SEQ ID NO. 16.

In another preferred example, the recombinant vector CD7BB-BL6-002 is formed by connecting TH69BB-002 to CD7-L-ER2.1 via T2A. The structural schematic diagram of the recombinant vector is as shown in (3) and (4) in B of Figure 5, and the amino acid sequence of T2A is as shown in SEQ ID NO. 16.

In another preferred example, the signal peptide in the vector is derived from CD8α.

In another preferred example, the amino acid sequence of the CD8α signal peptide in the vector is as shown in SEQ ID NO. 12.

In another preferred example, the signal peptide in the vector is derived from GM-CSF-R.

In another preferred example, the amino acid sequence of the GM-CSF-R signal peptide in the vector is as shown in SEQ ID NO. 13.

In another preferred example, the light chain and heavy chain of the scFv of the vector are linked via a connecting peptide (GGGGS)3, and the amino acid sequence of the connecting peptide is as shown in SEQ ID NO. 14.

In another preferred example, the light chain and heavy chain of the scFv of the vector are linked via a connecting peptide Whitlow, and the amino acid sequence of the connecting peptide is as shown in SEQ ID NO. 15.

In another preferred example, the nucleic acid molecules encoding the chimeric antigen receptor is connected to the nucleic acid molecule encoding the CD7 blocking molecule in the vector via an internal ribosome entry site (IRES) or a ribosomal codon skipping site.

In another preferred example, the internal ribosome entry site (IRES) in the vector is derived from encephalomyocarditis virus (EMCV) or enterovirus.

In another preferred example, the ribosomal codon skipping site in the vector comprises a 2A self-cleaving peptide, which can be selected from foot-and-mouth disease virus 2A peptide, equine rhinitis A virus 2A peptide, porcine teschovirus-1 2A peptide, or thosea asigna virus 2A.

In another preferred example, the 2A self-cleaving peptide in the vector is derived from T2A.

In another preferred example, the amino acid sequence of the T2A self-cleaving peptide in the vector is as shown in SEQ ID NO. 16.

In another preferred example, the 2A self-cleaving peptide in the vector is derived from F2A.

In another preferred example, the amino acid sequence of the F2A self-cleaving peptide in the vector is as shown in SEQ ID NO. 17.

The fifteenth aspect of the present invention provides the use of the recombinant vector as described in the fourteenth aspect in the preparation of an engineered immune cell.

The sixteenth aspect of the present invention provides a reagent combination, comprising: (1) the recombinant vector as described in the fifth aspect above, and (2) the vector as described in the eleventh aspect above.

The seventeenth aspect of the present invention provides the use of the reagent combination as described in the sixteenth aspect in the preparation of CAR-T or CAR-NK cells for treating CD7-positive hematological malignancies.

The eighteenth aspect of the present invention provides a reagent combination, comprising: (1) the vector as described in the fifth aspect above, and (2) a gene editing tool capable of knocking out the CD7 gene in cells.

In another preferred example, the gene editing tool is TALENs or CRISPR/cas9.

The nineteenth aspect of the present invention provides a sequence of the CD7-targeted antigen recognition domain in the chimeric antigen receptor, wherein the sequence is a scFv of an anti-CD7 monoclonal antibody TH69, or has at least 90% sequence identity to the scFv of the anti-CD7 monoclonal antibody TH69, and the amino acid sequence of the scFv of the anti-CD7 monoclonal antibody TH69 is as shown in SEQ ID NO. 8.

The twentieth aspect of the present invention provides a nucleic acid molecule encoding the sequence of the CD7-targeted antigen recognition domain in the chimeric antigen receptor as described in the nineteenth aspect.

In another preferred example, the coding sequence of the nucleic acid molecule as described in the twentieth aspect is TH69BB-002, and the nucleic acid molecule sequence of TH69BB-002 is as shown in SEQ ID NO. 18.

The twenty-first aspect of the present invention provides a recombinant vector, comprising the sequence of the nucleic acid molecule as described in the twentieth aspect.

In another preferred example, the vector is selected from a retrovirus, a lentivirus, or a transposon.

The twenty-second aspect of the present invention provides a reagent combination, comprising:
(1) the recombinant vector as described in the twenty-first aspect,
and (2) one selected from the following combination: the recombinant vector comprising the nucleic acid sequence as described in the eighth aspect, or a gene editing tool capable of knocking out the CD7 gene in cells.

The twenty-third aspect of the present invention provides the use of the reagent combination as described in the twenty-second aspect in the preparation of CAR-T or CAR-NK cells for treating CD7-positive hematological malignancies.

Compared with the prior art, the present invention has the beneficial effects as follows: in the present invention, the human CD7-L is used for substituting for an antibody sequence to serve as an antigen recognition domain of a CD7-specific CAR-T cell, and the advantage of using human CD7-L as the antigen recognition domain in targeted CD7 CAR is that cellular and humoral reactions produced by a host can be prevented, thereby achieving long-term survival and better efficacy of the CAR-T cells after being infused into the body.

CD7 is a transmembrane glycoprotein that is usually expressed in most peripheral T cells and NK cells, and the precursors thereof. The pathological T cells and NK cells themselves can express CD7 at high density. CD7-deficient T cells exhibit undisturbed development, homeostasis, and protective functions to a large extent. Since the impact of CD7 on the function of peripheral blood T cells is not significant, CD7 is a promising target for CAR-T cell therapy. Since both normal and diseased T cells can express CD7, two factors need to be considered when preparing chimeric antigen receptor (CAR) T cells: 1. genetically modifying normal T cells to express CD7-targeted chimeric antigen receptor (CAR-T) so as to kill CD7-positive diseased T cells; 2. blocking the expression of CD7 in CAR-T cells themselves to prevent CAR-T cells from fratricide due to mutual recognition. Therefore, according to the technical solution of the present invention, the normal T cells are genetically modified to express CD7-specific CAR, while the expression of CD7 in normal T cells is blocked. The applicant of the present invention has conducted numerous experiments, continuously adjusted and verified experimental parameters, and finally obtained the technical solution of the present invention. According to the technical solution of the present invention, the normal T cells can be genetically modified to express CD7-specific CAR, while the expression of CD7 in normal T cells can also be blocked, thereby achieving unexpected technical effects.

### Description of the Drawings

By reading the following detailed description made with reference to the drawings for non-limiting embodiments, the other features, objectives and advantages of the present invention will become more apparent:

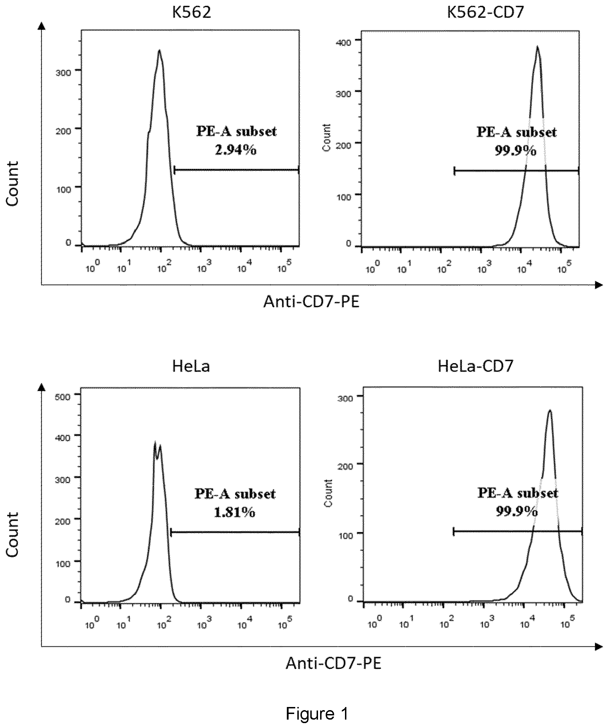

Figure 1 shows the construction of K562 and HeLa cell lines expressing CD7. The lentivirus vector carrying CD7 cDNA was used to transduce K562 and HeLa cell lines, and K562-CD7 and HeLa-CD7 were obtained by sorting using flow cytometry. The figure shows the expression of CD7 on K562 and HeLa cell lines by flow cytometry.

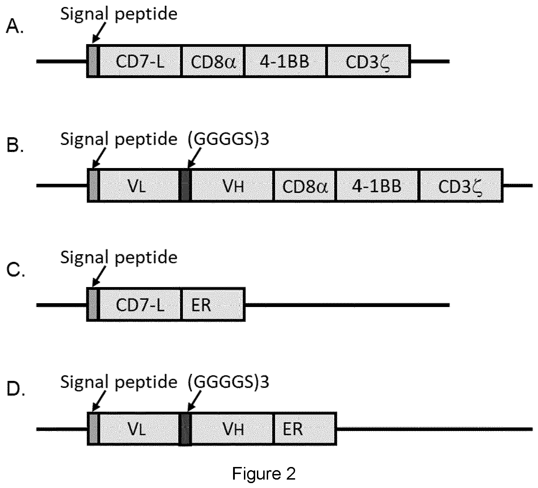

Figure 2 is the structural schematic diagram of the vector for CD7-CAR and CD7 blocking molecules. A and B are two second-generation CD7-specific CARs. Specifically, in A, the antigen recognition region of CD7BB-002 is CD7-L; and in B, the antigen recognition region of TH69BB-002 is the scFv of the monoclonal antibody TH69. Both CAR vectors have the same CD8a hinge and transmembrane region, 4-1BB co-stimulatory domain, and CD3ζ which is a T cell stimulatory domain. The CD8a signal peptide is used for the scFv of monoclonal antibody TH69, and the connecting peptide for linking variable regions of light chain (VL) and heavy chain (VH) is (GGGGS)3. C and D are two CD7 blocking molecules. Specifically, in C, the CD7 binding domain of CD7-L-ER2.1 is CD7-L; and in D, the CD7 binding domain of TH69-ER2.1 is the scFv of TH69, CD8α signal peptide is used therefor, and the connecting peptide for linking variable regions of light chain (VL) and heavy chain (VH) is (GGGGS)3; Two CD7 blocking molecules have the same endoplasmic reticulum (ER) retention domain.

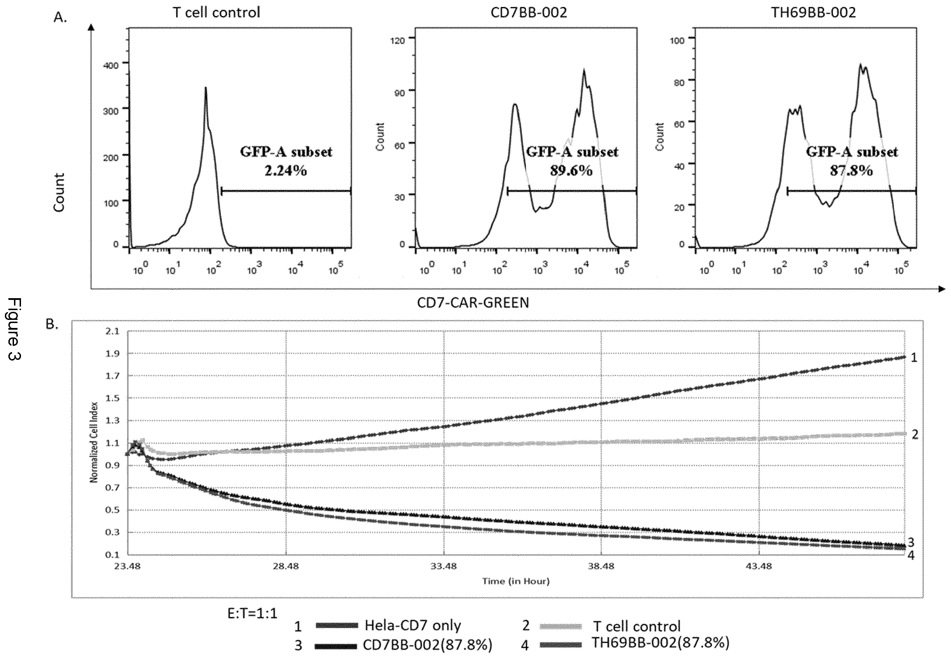

Figure 3 shows the schematic diagram of the results of flow cytometry and in vitro cytotoxicity experiment on CD7-CAR-T cells. A shows the expression of two CD7-targeted CARs, CD7BB-002 and TH69BB-002, on T cells detected by flow cytometry. B shows in vitro cytotoxicity experiments using the iCELLigence^{™} real-time cell analyser (Agilent Biosciences, Inc.). "T cell control" refers to untransduced T cells as a control, "CD7BB-002" refers to CAR-T cells expressing CD7BB-002, and "TH69BB-002" refers to CAR-T cells expressing TH69BB-002. The results show that both CAR-T cells are able to target CD7 so as to kill tumor cells. The detection reagent used in flow cytometry for CD7-CAR-T cells is CD7-CAR-GREEN, and the target cells used in the cellular cytotoxicity experiment in vitro are HeLa-CD7.
Figure 4 shows the schematic diagram of the results of blocking CD7 expression on the cell membrane using Intrablock^{™} CD7 expression blocking technology. The lentivirus vectors CD7-L-ER2.1 and TH69-ER2.1, which carry ligand CD7-L that can bind to CD7 or TH69 scFV cDNA and connected to the ER retention domain, are used to transduce K562-CD7 cell lines (A) or T cells (B), and flow cytometry was performed for CD7. The results show that both CD7 expression blocking molecules can effectively reduce the expression of CD7 on the cell surface.
Figure 5 is the structural schematic diagram of the CD7 CAR-targeted lentiviral vector constructed using Intrablock^{™} CD7 expression blocking technology. A and B are two CD7-CAR lentiviral vectors constructed using Intrablock^{™} CD7 expression blocking technology. Specifically, in A, for CD7BB-BL4-002, (1) is the schematic diagram of the CD7-targeted chimeric antigen receptor, and the antigen recognition region thereof is CD7-L; (2) is the schematic diagram of the CD7 blocking molecule, and the CD7 binding domain that plays a role in blocking CD7 expression is the scFv of the monoclonal antibody TH69. in B, for CD7BB-BL6-002, (3) is the schematic diagram of the CD7-targeted chimeric antigen receptor, and the antigen recognition region thereof is the scFv of the monoclonal antibody TH69; (4) is the schematic diagram of the CD7 blocking molecule, and the CD7 binding domain that plays a role in blocking CD7 expression is CD7-L. Two CAR vectors have the same CD8α hinge and transmembrane region, 4-1BB co-stimulatory domain, CD3ζ T cell stimulatory domain, and endoplasmic reticulum (ER) retention domain. The CD8α signal peptide is used for the scFv of monoclonal antibody TH69, and the connecting peptide for linking variable regions of light chain and heavy chain is (GGGGS)3.

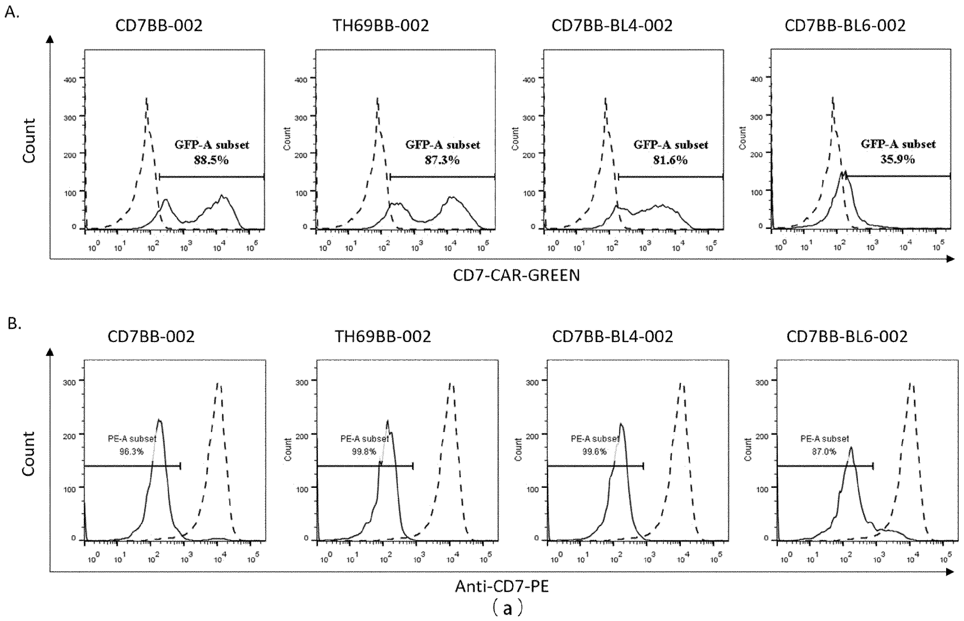

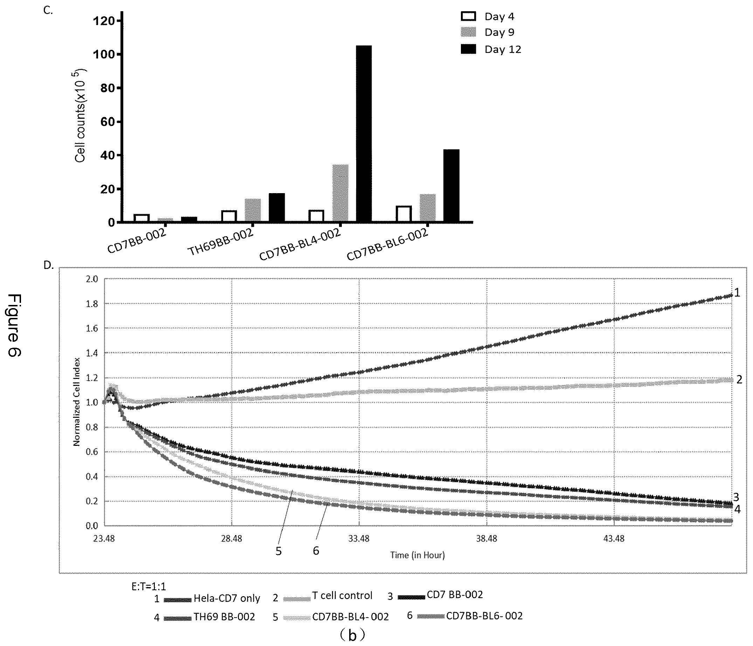

In Figure 6, CD7-CAR-T cells can effectively kill target cells without fratricide by using the Intrablock^{™} CD7 expression blocking technology. In (a), A shows the expression of four different CD7-targeted CARs on T cells detected by flow cytometry; B shows the expression of CD7 on the above-mentioned four different CAR-T cells detected by flow cytometry. The vectors used are: CD7BB-002, TH69BB-002, as well as CD7BB-BL4-002 and CD7BB-BL6-002 constructed using Intrablock^{™} CD7 expression blocking technology, respectively. The dashed line represents untransduced T cells as a control, and the solid line represents CAR-transduced T cells targeting CD7. In (b), C shows the observations of proliferation of the above-mentioned four different CAR-T cells cultured in vitro; D shows the cytotoxicity experiments in vitro using the iCELLigence^{™} real-time cell analyser (Agilent Biosciences, Inc.). T cell control refers to the untransduced T cells as a control, CD7BB-002, TH69BB-002, CD7BB-BL4-002, and CD7BB-BL6-002 are all CAR-T cells targeting CD7, wherein CD7BB-BL4-002 and CD7BB-BL6-002 are CD7-CAR-T cells that are prepared using Intrablock^{™} CD7 expression blocking technology. The detection reagent used in flow cytometry for CD7-CAR-T cells is CD7-CAR-GREEN, and the target cells used in the cellular cytotoxicity experiment in vitro are HeLa-CD7.

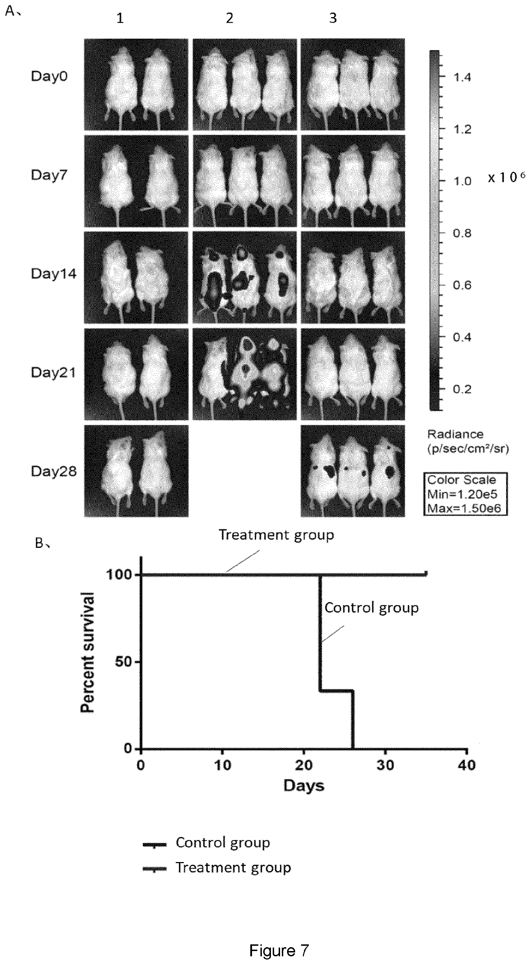

Figure 7 shows the results of the tumor cytotoxicity experiment in vivo of CD7-CAR-T cells prepared using Intrablock^{™} CD7 expression blocking technology. Female NSG mice were used, and injected with tumor cells (tumor cell lines carrying luciferase, CCRF-CEM-Luc, 5x10E5/mouse, i.v.) on day 0. A. 1 shows negative control group; 2 and 3 show T cell-infused control group and CD7-targeted CD7BB-BL4-002 CAR-T cell treatment group, respectively. After being injected with tumor cells, the mice were separately injected intravenously with T cells or CD7BB-BL4-002 CAR-T cells (8 x 10E6/mouse) on day 3, and then observed for luciferase imaging in vivo every 7 days. B shows the survival curves of the mice from T cell-infused control group and CD7BB-BL4-002 CAR-T cell treatment group.

### Detailed Description of Embodiments

The present invention is described in detail in combination with specific embodiments as below. The following embodiments will help those skilled in the art to further understand the present invention, but do not limit the present invention in any way. It should be noted that a person of ordinary skill in the art can also make various alterations and improvements without departing from the concept of the present invention of which shall fall within the scope of protection of the present invention.

In the present invention, the extracellular domain of SECTM1 (K12) is used as the antigen recognition domain of CD7, which is used to develop CAR-T cell therapies and immunotoxins for CD7-positive hematological malignancies. In this patent application, CD7-L is used for substituting for SECTM1 or K12. The CD7-L gene was initially identified at the 5' end of the CD7 gene on human chromosome 17. Human CD7-L protein is mainly expressed in the spleen, prostate, testis, small intestine, and peripheral blood leukocytes. CD7-L has one characteristic, that is, it encodes one transmembrane protein, and its extracellular domain is similar to the immunoglobulin-like domain. CD7-L was cloned in 2000 and found to be a binding protein of CD7.

In order to determine the binding activity of CD7-L protein, a fusion protein of the extracellular domain of CD7-L (amino acids 1-145) and the Fc portion of human IgG1 was used in previous studies. The experiments using flow cytometry show that binding activity of CD7-L-Fc fusion protein can be detected on human T cells and NK cells at high level. Several CD7-targeted antibodies block the binding of CD7-L-Fc fusion protein to cells to varying degrees. Conversely, CD7-L-Fc fusion protein can block the binding of these CD7 monoclonal antibodies to CD7, indicating that CD7-L-Fc can bind to CD7 receptors on cells. CD7-L-Fc fusion protein are radiolabeled and used in the experiments of binding activity to determine its affinity to Jurkat cells (human T-cell leukemia cell line) or KG-1 cells (human myelocytic leukemia cell line), both of which express CD7. The binding affinity (Ka) of CD7-L-Fc to human CD7 is approximately within 1 × 10⁸M⁻¹. Since CD7 is considered to be a good marker for T cell malignancies, the anti-human CD7 monoclonal antibodies have been used in previous studies in conjugation with ricin or saponin to produce immunotoxins.

Therefore, in this patent application, the extracellular domain of CD7-L is used for constructing CD7-targeted chimeric antigen receptor and CD7 blocking molecules for the development of CD7-CAR-T or CAR-NK cells to treat T cell malignancies. Additionally, in this patent application, the extracellular domain of CD7-L is conjugated to toxins, and these conjugates are used as immunotoxins against T cell malignancies, which may have a longer half-life because of their lower immunogenicity than antibody-based conjugates.

Example 1. CAR-T cells in which CD7-L is used as the antigen recognition domain can effectively recognize CD7 tumor antigens.

In this Example, two second-generation CAR lentiviral vectors, CD7BB-002 and TH69BB-002 (Figures 2A and 2B), in which CD7-L or the scFv of the CD7-specific monoclonal antibody TH69 was used as the antigen recognition region were constructed. CAR-T cells were obtained by transducing with the CAR lentiviral vector, and subjected to flow cytometry using flow cytometry reagent (CD7-CAR-GREEN) produced by CD7 and the reporter gene eGFP-fused protein for CD7-CAR-T cells (Figure 3A). The results show that CD7-CAR-GREEN can be used to effectively detect the two CD7-targeted CAR-T cells, indicating that both CD7-L and the scFv of the monoclonal antibody TH69 can serve as the CD7-specific antigen recognition region of CAR-T cells.

Example 2. CD7-targeted CAR-T cells can effectively kill CD7-positive tumor cells.

In this Example, the lentivirus vector carrying CD7 cDNA was used to transduce K562 and HeLa cell lines, and K562-CD7 and HeLa-CD7 cell lines expressing CD7 were obtained by sorting using flow cytometry (Figure 1). Two CD7-targeted CAR-T cells, CD7BB-002 and TH69BB-002, were obtained by transducing with CAR lentiviral vectors, and subjected to cytotoxicity experiments in vitro using the iCELLigence^{™} real-time cell analyser (Agilent Biosciences, Inc.) (Figure 3B). The results show that CD7BB-002 in which CD7-L is used as the antigen recognition region and TH69BB-002 in which the scFv of the monoclonal antibody TH69 is used as the antigen recognition region can effectively recognize CD7 antigen and have the same effect in terms of killing CD7-positive HeLa-CD7 target cells.

Example 3. Intrablock^{™} CD7 expression blocking technology can effectively block the expression of CD7 on the cell membrane.

In this Example, lentiviral vectors, CD7-L-ER2.1 and TH69-ER2.1, with CD7-L or the scFv of the CD7-specific monoclonal antibody TH69 as the CD7 binding domain and connected to the ER retention domain were firstly constructed (Figures 2C and 2D). K562-CD7 cells lines or primary T cells were transduced with lentiviral vectors, and flow cytometry was performed to evaluate the CD7 expression blocking technology. The results show that TH69-ER2.1 and CD7-L-ER2.1 can effectively block the expression of CD7 on K562-CD7 cell lines (Figure 4A) or T cells (Figure 4B), indicating that TH69-ER2.1 and CD7-L-ER2.1 can bind to CD7 in cells and retain it in the endoplasmic reticulum. Therefore, this Intrablock^{™} CD7 expression blocking technology can be used to block the expression of CD7 on cells and prevent CD7-targeted CAR-T cells from fratricide.

Example 4. Intrablock^{™} CD7 expression blocking technology can be used to prevent CD7-CAR-T cells from fratricide, and the CD7-positive target cells can be effectively killed.

In this Example, CAR lentiviral vectors, CD7BB-BL4-002 and CD7BB-BL6-002, which have Intrablock^{™} CD7 expression blocking function, and also target CD7, were firstly constructed (Figures 5A and 5B). Since T cells used for preparing CAR-T cells express CD7 themselves, the behavior of fratricide occurs during the preparation of CD7-targeted CAR-T cells, making it difficult to prepare CAR-T cells. As shown in Figure 6, two CD7-targeted CAR-T cells, CD7BB-002 and TH69BB-002, can be effectively recognized by CD7-CAR-GREEN (Figure 6A) and can effectively kill CD7-positive HeLa-CD7 target cells (Figure 6D). However, severe behavior of fratricide occurs during in vitro cultivation of the two CD7-targeted CAR-T cells, causing difficulties in the expansion and preparation of CAR-T cells in vitro (Figure 6C). CD7BB-BL4-002 and CD7BB-BL6-002 lentiviral vectors targeting CD7 were constructed using Intrablock^{™} CD7 expression blocking technology described in Example 3 and used to prepare CAR-T cells. The two CAR-T cells, CD7BB-BL4-002 and CD7BB-BL6-002, prepared using the Intrablock^{™} CD7 expression blocking technology, can be effectively recognized by CD7-CAR-GREEN (Figure 6A), and maintain the ability for killing HeLa-CD7 target cells (Figure 6D); in addition, this technology can block the expression of CD7 (Figure 6B) and prevent CAR-T cells from fratricide during preparation (Figure 6C), thereby making it possible to expand and prepare CD7-targeted specific CAR-T cells in vitro.

Example 5: Verification of the tumor-killing function in vivo of CD7-CAR-T cells prepared by using Intrablock^{™} CD7 expression blocking technology in animal models.

In this Example, CD7BB-BL4-002 CAR-T cells prepared by using Intrablock^{™} CD7 expression blocking technology were used for tumor cytotoxicity experiments in vivo (Figure 7). Female NSG mice aged 6-8 weeks were used, and injected with tumor cell lines (CCRF-CEM-Luc, 5 x 10E5/mouse, i.v.) carrying luciferase via tail vein on day 0. After being injected with tumor cells, the mice were separately injected intravenously with T cells (T cell-infused control group) or CD7BB-BL4-002 CAR-T cells (8 x 10E6/mouse) (CAR-T cell treatment group) on day 3. The mice were observed for luciferase imaging in vivo every 7 days after day 0. The results show that CD7BB-BL4-002 CAR-T cells prepared by using Intrablock^{™} CD7 expression blocking technology can effectively kill tumors in such mouse tumor models and prolong the survival of mice from the CAR-T cell treatment group (as shown in A and B of Figure 7).

Compared with the prior art, the present invention has the beneficial effects as follows: in the present invention, the human CD7-L is used for substituting for an antibody sequence to serve as an antigen recognition domain of a CD7-specific CAR-T cell, and the advantage of using human CD7-L as the antigen recognition domain in targeted CD7 CAR is that cellular and humoral reactions produced by a host can be prevented, thereby achieving long-term survival and better efficacy of the CAR-T cells after being infused into the body. CD7 is a transmembrane glycoprotein that is usually expressed in most peripheral T cells and NK cells, and the precursors thereof. The pathological T cells and NK cells themselves can express CD7 at high density. CD7-deficient T cells exhibit undisturbed development, homeostasis, and protective functions to a large extent. Since the impact of CD7 on the function of peripheral blood T cells is not significant, CD7 is a promising target for CAR-T cell therapy. Since both normal and diseased T cells can express CD7, two factors need to be considered when preparing chimeric antigen receptor (CAR) T cells: 1. genetically modifying normal T cells to express CD7-targeted chimeric antigen receptor (CAR-T) so as to kill CD7-positive diseased T cells; 2. blocking the expression of CD7 in CAR-T cells themselves to prevent CAR-T cells from fratricide due to mutual recognition. Therefore, according to the technical solution of the present invention, the normal T cells are genetically modified to express CD7-specific CAR, while the expression of CD7 in normal T cells is blocked. The applicant of the present invention has conducted numerous experiments, continuously adjusted and verified experimental parameters, and finally obtained the technical solution of the present invention. According to the technical solution of the present invention, the normal T cells can be genetically modified to express CD7-specific CAR, while the expression of CD7 in normal T cells can also be blocked, thereby achieving unexpected technical effects.

The specific embodiments of the present invention are described above. It should be understood that the present invention is not limited to the specific implementation described above, and various alterations or modifications may be made by a person skilled in the art within the scope of the claims, which does not affect the essential contents of the present invention. Intrablock is a trademark and does not constitute any limitation or restriction to the technical solution of the present invention. In the case of no conflict, the embodiments in the present application and the features in the embodiments can be arbitrarily combined with each other.

## Claims

1. An engineered immune cell comprising a polynucleotide sequence encoding a chimeric antigen receptor, wherein the chimeric antigen receptor comprises a CD7-targeted antigen recognition domain.

2. The engineered immune cell of claim 1, wherein the chimeric antigen receptor further comprises a hinge and transmembrane domain, an intracellular co-stimulatory domain, and an intracellular primary stimulatory domain.

3. The engineered immune cell of claim 2, wherein the hinge and transmembrane domain in the chimeric antigen receptor comprises at least one amino acid sequence selected from the following group: amino acid sequences of α and β chains from T cell receptors, CD3δ, CD3ε, CD3γ, CD3ζ chain, CD4, CD5, CD8α, CD8β, CD9, CD16, CD22, CD28, CD32, CD33, CD34, CD35, CD37, CD45, CD64, CD80, CD86, CD137, ICOS, CD154, FAS, FGFR2B, OX40, or VEGFR2.

4. The engineered immune cell of claim 2, wherein the intracellular co-stimulatory domain in the chimeric antigen receptor comprises at least one selected from the following group: CD2, CD4, CD5, CD8α, CD8β, CD27, CD28, CD30, CD40, 4-1BB (CD137), ICOS, OX40, LIGHT (CD258) or NKG2C.

5. The engineered immune cell of claim 2, wherein the intracellular primary stimulatory domain in the chimeric antigen receptor comprises at least one selected from the following group: CD3δ, CD3ε, CD3γ, CD3ζ, FcRβ, FcRy, CD5, CD66d, CD22, CD79a or CD79b.

6. The engineered immune cell of claim 1, wherein the CD7-targeted antigen recognition domain in the chimeric antigen receptor is a part or all of the sequences of a human CD7-L extracellular domain, or has at least 90% sequence identity to the human CD7-L extracellular domain, and the amino acid sequence of the human CD7-L extracellular domain is as shown in SEQ ID NO. 5.

7. The engineered immune cell of claim 6, wherein the engineered immune cell further comprises a CD7 blocking molecule, which can prevent the transport and expression of the CD7 protein on the cell surface.

8. The engineered immune cell of claim 7, wherein the CD7 blocking molecule prevents the CD7 protein from being transported to the cell surface specifically by connecting a CD7 binding domain to an intracellular anchoring domain.

9. The engineered immune cell of claim 8, wherein the intracellular anchoring domain is an amino acid sequence of an endoplasmic reticulum retention domain, a Golgi complex retention domain, or a proteasome localization domain.

10. The engineered immune cell of claim 8, wherein the CD7 binding domain is a part or all of the sequences of a human CD7-L extracellular domain, or is a protein having at least 90% sequence identity to the human CD7-L extracellular domain, and the amino acid sequence of the human CD7-L extracellular domain is as shown in SEQ ID NO. 5.

11. The engineered immune cell of claim 8, wherein the CD7 binding domain is a scFv of an anti-CD7 monoclonal antibody TH69.

12. The engineered immune cell of claim 1, wherein the CD7-targeted antigen recognition domain in the chimeric antigen receptor is a scFv of an anti-CD7 monoclonal antibody TH69, or has at least 90% sequence identity to the scFv of the anti-CD7 monoclonal antibody TH69, and the amino acid sequence of the scFv of the anti-CD7 monoclonal antibody TH69 is as shown in SEQ ID NO. 8.

13. The engineered immune cell of claim 12, wherein the engineered immune cell further comprises a CD7 blocking molecule, which can prevent the transport and expression of the CD7 protein on the cell surface.

14. The engineered immune cell of claim 12, wherein the CD7 blocking molecule prevents the CD7 protein from being transported to the cell surface by connecting a CD7 binding domain to an intracellular anchoring domain.

15. The engineered immune cell of claim 14, wherein the intracellular anchoring domain is an amino acid sequence of an endoplasmic reticulum retention domain, a Golgi complex retention domain, or a proteasome localization domain.

16. The engineered immune cell of claim 14, wherein the CD7 binding domain is a part or all of the sequences of a human CD7-L extracellular domain, or has at least 90% sequence identity to the human CD7-L extracellular domain, and the amino acid sequence of the human CD7-L extracellular domain is as shown in SEQ ID NO. 5.

17. The engineered immune cell of claim 6 or 12, wherein the engineered immune cells are deprived of the gene expression of CD7 through gene knockout technology.

18. The engineered immune cell of claim 17, wherein the gene editing tool used for the knockout technology is TALENs or CRISPR/cas9.

19. The engineered immune cell of any one of claims 1-16 and 18, wherein the cell is a T cell, a γδT cell, a NK or NKT cell, and a T cell, a γδT cell, a NK or NKT cell that induces differentiation of a pluripotent stem cell.

20. A CD7-targeted antigen recognition domain in the chimeric antigen receptor, wherein the sequence of the antigen recognition domain comprises a part or all of the sequences of a human CD7-L extracellular domain, or has at least 90% sequence identity to the human CD7-L extracellular domain, and the amino acid sequence of the human CD7-L extracellular domain is as shown in SEQ ID NO. 5.

21. The use of the CD7-targeted antigen recognition domain of claim 20 in the preparation of an immunotoxin for CD7-positive hematological malignancies.

22. A nucleic acid molecule encoding the CD7-targeted antigen recognition domain in the chimeric antigen receptor of claim 20.

23. The nucleic acid molecule encoding the CD7-targeted antigen recognition domain of claim 22, wherein the coding sequence of the nucleic acid molecule is CD7BB-002, and the nucleic acid molecule sequence of CD7BB-002 is as shown in SEQ ID NO. 9.

24. A recombinant vector, comprising the nucleic acid molecule encoding the CD7-targeted antigen recognition domain in the chimeric antigen receptor of claim 22.

25. The recombinant vector of claim 24, wherein the vector is selected from a retrovirus, a lentivirus, or a transposon.

26. The use of the recombinant vector of claim 24 in the preparation of an engineered immune cell.

27. A CD7 blocking molecule for the preparation of the engineered immune cell of any one of claims 1-16, wherein the CD7 binding domain of the CD7 blocking molecule comprises a part or all of the sequences of a human CD7-L extracellular domain, or is a protein having at least 90% sequence identity to the human CD7-L extracellular domain.

28. A nucleic acid sequence encoding the CD7 blocking molecule of claim 27.

29. The nucleic acid sequence encoding the CD7 blocking molecule of claim 28, wherein the nucleic acid sequence is CD7-L-ER2.1, and the nucleic acid molecule sequence of CD7-L-ER2.1 is as shown in SEQ ID NO. 10.

30. A CD7 blocking molecule for the preparation of the engineered immune cell of any one of claims 1-16, wherein the CD7 binding domain of the CD7 blocking molecule is a scFv of an anti-CD7 monoclonal antibody TH69, or has at least 90% sequence identity to the scFv of the anti-CD7 monoclonal antibody TH69.

31. A nucleic acid sequence encoding the CD7 blocking molecule of claim 30, wherein the coding sequence of the nucleic acid molecule of the CD7 blocking molecule is TH69-ER2.1, and the nucleic acid molecule sequence of TH69-ER2.1 is as shown in SEQ ID NO. 11.

32. A recombinant vector, comprising the nucleic acid sequence of claim 29 or 31.

33. The recombinant vector of claim 32, wherein the vector is selected from a retrovirus, a lentivirus, or a transposon.

34. The use of the recombinant vector of claim 32 in the preparation of an engineered immune cell.

35. A nucleic acid molecule, comprising:
(1) a nucleic acid sequence encoding the sequence of the antigen recognition domain of claim 20;
and (2) a nucleic acid sequence encoding the CD7 blocking molecule of claim 27 or claim 30.

36. A recombinant vector, comprising the nucleic acid molecule of claim 35.

37. The recombinant vector of claim 36, wherein the vector is selected from a retrovirus, a lentivirus, or a transposon.

38. The recombinant vector of claim 36, wherein the nucleic acid molecule encoding the chimeric antigen receptor is connected to the nucleic acid molecule encoding the CD7 blocking molecule in the vector via an internal ribosome entry site or a ribosomal codon skipping site.

39. The recombinant vector of claim 36, wherein the internal ribosome entry site in the vector is derived from encephalomyocarditis virus or enterovirus.

40. The recombination vector of claim 36, wherein the ribosomal codon skipping site in the vector comprises a 2A self-cleaving peptide, which can be selected from foot-and-mouth disease virus 2A peptide, equine rhinitis A virus 2A peptide, porcine teschovirus-1 2A peptide, or thosea asigna virus 2A.

41. The use of the recombinant vector of claim 36 in the preparation of an engineered immune cell.

42. A reagent combination, comprising: (1) the recombinant vector of claim 24, and (2) the recombinant vector of claim 32.

43. The use of the reagent combination of claim 42 in the preparation of CAR-T or CAR-NK cells for treating CD7-positive hematological malignancies.

44. A reagent combination, comprising: (1) the recombinant vector of claim 24, and (2) a gene editing tool capable of knocking out the CD7 gene in cells.

45. The reagent combination of claim 44, wherein the gene editing tool is TALENs or CRISPR/cas9.

46. A sequence of the CD7-targeted antigen recognition domain in the chimeric antigen receptor, wherein the sequence is a scFv of an anti-CD7 monoclonal antibody TH69, or has at least 90% sequence identity to the scFv of the anti-CD7 monoclonal antibody TH69, and the amino acid sequence of the scFv of the anti-CD7 monoclonal antibody TH69 is as shown in SEQ ID NO. 8.

47. A nucleic acid molecule encoding the sequence of the CD7-targeted antigen recognition domain in the chimeric antigen receptor of claim 46.

48. The nucleic acid molecule of claim 47, wherein the coding sequence of the nucleic acid molecule is TH69BB-002, and the nucleic acid molecule sequence of TH69BB-002 is as shown in SEQ ID NO. 18.

49. A recombinant vector, comprising the sequence of the nucleic acid molecule of claim 47.

50. The recombinant vector of claim 49, wherein the vector is selected from a retrovirus, a lentivirus, or a transposon.

51. A reagent combination, comprising:
(1) the recombinant vector of claim 49,
and (2) one selected from the following combination: a recombinant vector comprising the nucleic acid sequence of claim 29, or a gene editing tool capable of knocking out the CD7 gene in cells.

52. The use of the reagent combination of claim 51 in the preparation of CAR-T or CAR-NK cells for treating CD7-positive hematological malignancies.
